(19) **Europäisches Patentamt / European Patent Office / Office européen des brevets**

(11) **EP 3 968 220 B1**

(12) **EUROPEAN PATENT SPECIFICATION**

(45) Date of publication and mention
of the grant of the patent:
**09.04.2025 Bulletin 2025/15**

(21) Application number: **21199275.5**

(22) Date of filing: **02.02.2010**

(51) International Patent Classification (IPC):
*A61B 8/08* (2006.01)    *A61B 5/103* (2006.01)
*A61B 5/00* (2006.01)    *A61B 5/11* (2006.01)
*A61B 8/00* (2006.01)    *A61B 34/10* (2016.01)
*A61B 34/20* (2016.01)    *G06N 3/084* (2023.01)
*G16H 40/63* (2018.01)    *G16H 50/50* (2018.01)

(52) Cooperative Patent Classification (CPC):
G16H 40/63; A61B 5/0004; A61B 5/002;
A61B 5/1036; A61B 5/1038; A61B 5/1114;
A61B 5/1121; A61B 5/1127; A61B 5/4528;
A61B 5/4533; A61B 5/4585; A61B 5/6807;
A61B 5/6828; A61B 5/6831; A61B 5/6833; (Cont.)

(54) **A METHOD OF EVALUATING A PHYSIOLOGICAL CONDITION OF BODILY TISSUE**

VERFAHREN ZUR BEWERTUNG EINES PHYSIOLOGISCHEN ZUSTANDS VON KÖRPERGEWEBE

PROCÉDÉ PERMETTANT D'ÉVALUER L'ÉTAT PHYSIOLOGIQUE D'UN TISSU CORPOREL

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **02.02.2009 US 364267**

(43) Date of publication of application:
**16.03.2022 Bulletin 2022/11**

(62) Document number(s) of the earlier application(s) in accordance with Art. 76 EPC:
**10736584.3 / 2 391 971**

(73) Proprietor: **Jointvue, LLC
Knoxville, Tennessee 37996-0001 (US)**

(72) Inventors:
• **Wasielewski, Ray C.**
  **New Albany, 43054 (US)**
• **Mahfouz, Mohamed Rashwan**
  **Knoxville, 37922 (US)**
• **Komistek, Rick**
  **Knoxville, 37934 (US)**

(74) Representative: **HGF
HGF Limited
1 City Walk
Leeds LS11 9DX (GB)**

(56) References cited:
**US-A1- 2007 249 967    US-A1- 2009 018 445**

**(Cont. next page)**

EP 3 968 220 B1

(52) Cooperative Patent Classification (CPC): (Cont.)
**A61B 5/7267; A61B 5/743; A61B 5/7435;**
**A61B 8/0858; A61B 8/0875; A61B 8/4227;**
**A61B 8/4254; A61B 8/4263; A61B 8/5223;**
**G06N 3/084; G16H 50/50;** A61B 5/0002;
A61B 5/112; A61B 5/389; A61B 8/4236;

A61B 8/4472; A61B 8/4477; A61B 8/466;
A61B 8/467; A61B 8/483; A61B 8/5238;
A61B 2034/105; A61B 2034/2048; A61B 2034/2051;
A61B 2562/02; A61B 2562/0204; A61B 2562/0219;
A61B 2562/0247; A61B 2562/046; G16H 20/30

**Description**

**Field of the Invention**

The present invention is defined in the claims and relates to diagnosis of bodily abnormalities, and more particularly, to devices and methods for evaluating the physiological condition of bodily tissue, such as bodily joints, to discern whether abnormalities exist and the extent of any abnormalities. While the exemplary embodiments disclosed herein are utilized and discussed with respect to a human knee joint, it is to be understood that other joints and bodily tissues may be similarly diagnosed.

BACKGROUND

In humans, the knee joint is functionally controlled by a mechanical system governed by three unique types of forces (1) active forces resulting from motion, such as those resulting from muscle flexing or relaxing, (2) constraining forces that constrain motion, such as those resulting from ligaments being in tension, and (3) interaction forces that resist motion, such as those acting upon bones In addition to these three types of forces, the soft tissue in the knee joint complex (e.g., cartilage and meniscus) produce a dampening effect distributing the compressive loads acting on the knee joint.

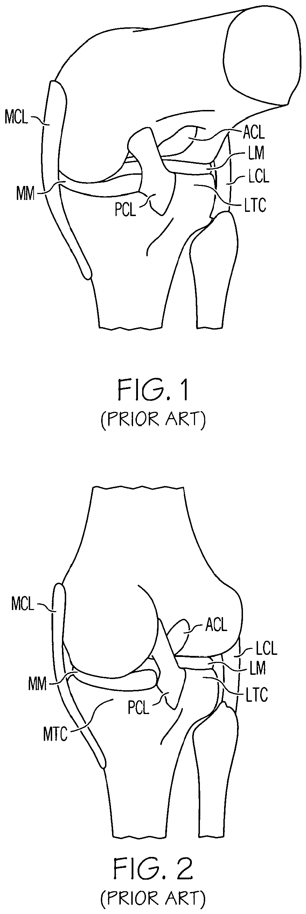

Knee joint motions are stabilized primarily by four ligaments, which restrict and regulate the relative motion between the femur, tibia, and patella. These ligaments are the anterior cruciate ligament (ACL), the posterior cruciate ligament (PCL), the medial collateral ligament (MCL), the lateral collateral ligament (LCL), and the patellar ligament, as shown in FIGS. 1 and 2. An injury to any of these ligaments or other soft-tissue structures can cause detectable changes in knee kinematics and the creation of detectable vibrations representative of the type of knee joint injury and the severity of the injury. These visual (knee kinematics) and auditory (vibrations) changes are produced by the bones while moving in a distorted kinematic pattern, and they differ significantly from the look and sound of a properly balanced knee joint moving through a range of motion. US20070249967 discloses a method for performing computer-assisted orthopaedic surgery which includes the step of producing and displaying three-dimensional geometrical models of first and second bones forming a joint. US2009018445 discloses an ultrasonic bone motion tracking system.

INTRODUCTION TO THE DISCLOSURE

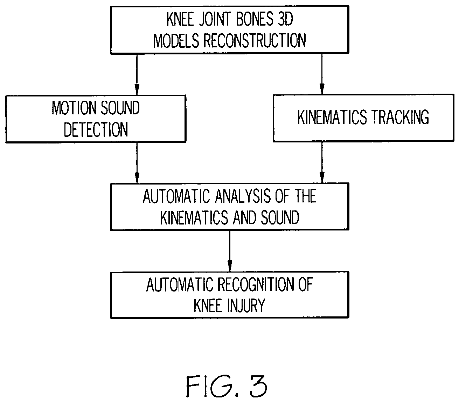

The invention is defined in claim 1, with further aspects of the invention in the dependent claims. There is provided in this disclosure a method of evaluating a physiological condition of bodily tissue in accordance with the present claims. The exemplary embodiments of the present disclosure include a diagnostic system for mammalian bodies to determine what type of injury, if any, exists and extent of any such injury using kinematic data and/or sound data that is patient specific. In particular, an exemplary method and embodiment are directed to a knee joint diagnostic system for automatically determining whether an injury exists, what the injury is (i.e., diagnosis), and the extent to which ligaments, muscles, bones, meniscus, and cartilage may be affected by the injury by gathering and analyzing patient specific kinematic data of the knee joint, while also gathering and analyzing the pattern and spatial distribution of sound(s) (i.e., vibrations) produced by movement of the patient's knee joint. A low level exemplary process flow diagram for this exemplary method is shown in FIG. 3.

An exemplary method of the present disclosure includes generating a patient-specific 3D tissue model of the bodily area in question (e.g., the bones of knee joint), obtaining patient-specific kinematic and sound data as the bodily area is taken through a range of motion, and finally analyzing the kinematic and sound data to discern whether an injury is present and, if so, the extent of any injury. In exemplary form, patient-specific 3D tissue models of the distal femur, proximal tibia, and the patella are constructed using conventional imaging technologies, such as computed tomography (CT) scans, fluoroscopy, magnetic resonance imaging (MRI) scans, X-rays, and the like. Alternatively, the exemplary embodiments of the present disclosure provide an alternative to conventional imaging technologies by utilizing A-mode ultrasound echo morphing technology to generate data necessary to construct 3D tissue models. After the patient-specific 3D tissue models have been generated, patient-specific kinematic data is gathered and evaluated for the motions of the femur, tibia, and patella. By way of example, this kinematic data may be obtained using the same A-mode ultrasound technology as is utilized to generate the data necessary to create the patient-specific 3D tissue models. In addition, patient-specific sound data is generated using accelerometers to monitor the knee joint while the joint is taken through a range of motion in a loaded, real-world condition. Finally, the kinematic data and sound data are analyzed to determine the most accurate diagnosis, including whether an injury exists and the extent of any such injury. In exemplary form, the kinematic data and sound data are analyzed by a neural network having actual kinematic and sound data correlated to correct diagnoses. The neural network is constantly updated with new data for cases where kinematic and sound data was obtained and the correct diagnosis was verified by in vivo assessment. Accordingly, as the neural network grows with more data, the precision of the diagnoses is correspondingly increased.

In exemplary form, the sound data and kinematic data may be obtained at the same time using a single data acquisition device. Moreover, the sound data and kinematic data are obtained in real time as the bodily area in question is taken through a range of motion. In a further exemplary embodiment, if the sound and kinematic data is acquired in a physician's office, the data may be displayed in real-time on a split screen monitor. If, however, the data is acquired outside of the doctor's office, a recording device and memory may be utilized to record the data in a time synced manner. In a yet a further exemplary embodiment, the patient may be given an actuator that is operative to note the general time frame within which the patient experienced a particular pain or severe pain to allow a diagnosis that correlates pain experienced by the patient and the kinematics and sound occurring at precisely or generally the same time.

As discussed above, the kinematic data and sound data generated on a patient-specific basis is analyzed by a trained neural network in order to provide an automated output as to the existence of an injury, the type of injury, and the severity of the injury. This neural network may be accessible via the internet or may reside on a physician's local computer. In addition, or in the alternative, patient-specific sound and kinematic data may be analyzed by a physician to make or verify the diagnosis with or without the aid of the neural network.

Using the exemplary methods and devices as disclosed herein, a physician may diagnose a bodily injury without requiring experimental surgery or requiring exposure of the patient to radiation of any form, such as X-rays or fluoroscopy. In addition, the data taken regarding each patient is continuous through a range of motion, in contrast to radiographic modalities that generate images at distinct points with significant range of motions gaps. In addition, data taken in accordance with the exemplary methods and devices disclosed herein also contracts data taken by a magnetic resonance imaging machine, not only because the data taken is continuous along the range of motion, but also because the bodily portion evaluated is acting under loaded conditions in a dynamic environment.

BRIEF DESCRIPTION OF THE DRAWINGS

FIG 1 is a posterior view of a human knee joint in a flexed position.

FIG 2 is a posterior view of a human knee joint in an extended position.

FIG 3 is an exemplary process flow diagram using exemplary methods within the scope of the present disclosure.

FIG 4 is a schematic diagram of the modules of an exemplary diagnostic system.

FIG 5 is frontal view of a knee brace in accordance with the instant disclosure that is mounted proximate a human knee joint.

FIG 6 is a graphical depiction of an exemplary shoe having a sensor array in its insole that is operative to detect pressure forces applied by a human foot under loaded conditions.

FIG 7 is an exemplary ultrasound transducer wand for use with the instant invention.

FIG 8 is a schematic diagram of an ultra wide band transmitter, where a baseband pulse is upconverted, amplified, filtered, and transmitted via an omnidirectional antenna.

FIG 9 is a schematic diagram of an ultra wide band receiver, where the signal is bandpass filtered, amplified, downconverted, low-pass filtered, sub-sampled, and converted to digital Leading-edge detection is performed on the Field Programmable Gate Array (FPGA), and the final time-difference-of-arrival (TDOA) calculation is performed on a computer.

FIG 10 is a 3D coordinate representation showing how the ultra wide band transmitter position in such a 3D coordinate system is determined.

FIG 11 is a diagram depicting the similarities between using ultra wide band receivers and transmitters for 3D positioning, in comparison to how a GPS receiver and satellites work to provide global positioning.

FIG 12 is a 3-D dynamic error plot while collecting 1000 data points and showing the error in the position of the x, y, and z axes compared to an optical reference system.

FIG 13 is a screen shot of a user interface of a computer where the 3D reference points taken of the patient's bone are applied to a default bone model, thereby creating a patient-specific bone model.

FIG 14 is a 3D representation of an exemplary patient-specific bone model that incorporates the distal femur, proximal tibia, and patella.

FIG 15 is an elevated, profile view of an exemplary knee brace in accordance with the instant disclosure.

FIG 16 is a schematic of the overall classification system flow chart.

FIG 17 is a schematic representation of an exemplary neural network classifier.

FIG 18 is an exemplary process flow for training an exemplary neural network.

FIG 19 is an exemplary process flow for knee deficiency diagnosis using a trained neural network.

DETAILED DESCRIPTION

The exemplary embodiments of the present disclosure are described and illustrated below to encompass diagnosis of bodily abnormalities and, more particularly, devices and methods for evaluating the physiological condition of bodily tissue (such as joints) to discern whether abnormalities exist and the extent of any abnormalities. Of course, it will be apparent to those of ordinary skill in the art that the exemplary embodiments discussed below are merely examples and may be reconfigured without departing from the scope of the present claims. By way of example, the exemplary embodiments disclosed herein are described with respect to diagnosing a knee joint injury. Nevertheless, the exemplary embodiments may be utilized to diagnose other bodily tissue injuries (such as a hip joint injury or a bone fracture), as the knee joint is merely exemplary to facilitate an understanding of the embodiments disclosed.

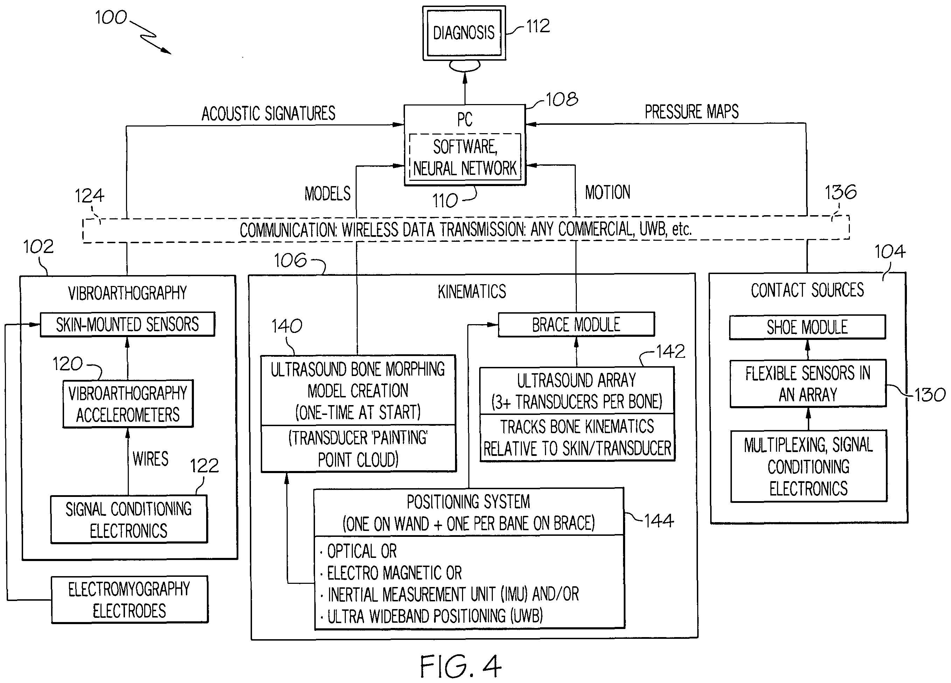

Referencing FIG. 4, a first exemplary diagnostic system 100 includes a plurality of modules 102, 104, 106 that output data to a computer 108 for data processing by way of a neural network 110. The data processing, as will be discussed in more detail below, provides a visual output, an audible output, and a diagnosis by way of a visual display 112. Again, as will be discussed below, the diagnosis includes detection of an injury, as well as information pertaining to the severity of the injury. But before the output from the system 100 can be fully explained, the modules 102, 104, 106 and the functionality of each will be described initially.

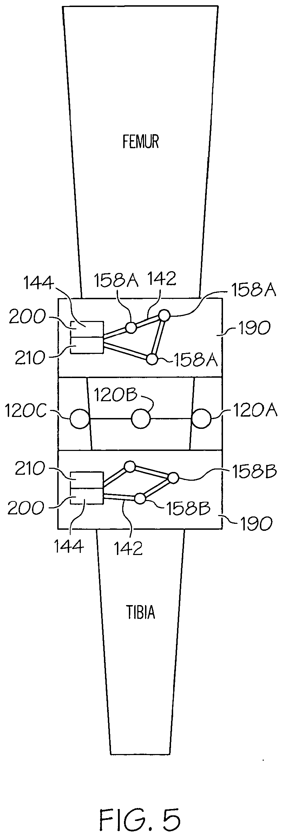

Referring to FIGS. 4 and 5, the system 100 includes a vibroarthography module (VM) 102 comprising a plurality of accelerometers 120 that are utilized to detect sound, specifically vibrations occurring as a result of motion of the knee joint. In this exemplary VM 102, the accelerometers are mounted directly to the skin or external tissue surface of a patient in order to detect bone and soft tissue interaction. An intervening adhesive is utilized between the accelerometers 120 and the patient's external tissue surface in order to secure the accelerometers in a fixed position.

In the context of a knee joint, an exemplary VM module includes three accelerometers 120, where one accelerometer 120A is mounted on the medial side of the knee joint, while a second accelerometer 120B is mounted on the lateral side of the knee joint, while a third accelerometer 120C is mounted on the front side of the knee joint proximate the patella. In this exemplary embodiment, the accelerometers 120 are mounted to the patient so that each lies along a common plane, though each could be mounted so as not to lie along a common plane It should also be understood, however, that more than three accelerometers and less than three accelerometers may be utilized to detect sound generated by dynamic interactions of the tissues against one another.

Each of the accelerometers 120 is in communication with signal conditioning circuits 122 associated with the vibroarthography module (VM) 102. The accelerometers 120 are operative to detect sound, specifically vibrations, and output the sound detected in the form of frequency data measured in Hertz to the conditioning circuits. This frequency data is processed by the conditioning circuits 122 and communicated to the computer 108 as digital frequency data. At the same time as the accelerometers 120 are generating frequency data, the conditioning circuits 122 may include a clock that time stamps the frequency data generated. As will be discussed in more detail below, correlating the frequency data with time provides a constant against which all of the detected data can be compared against on a relative scale.

As will be discussed in more detail hereafter, the interaction between bodily tissue (e.g., bone against cartilage, bone against bone) in a dynamic environment creates certain vibrations that are indicative of the condition or state of health of the joint. Even the healthiest and youngest joints create vibrations of some sort. However, joints that exhibit degradation, through wear or injury, will exhibit vibrations much more pronounced and amplified over those of a healthy joint. The exemplary embodiment of the disclosure takes advantage of the sound, such as vibrations, exhibited by the joint during a range of motion to diagnose the condition of the joint without requiring an invasive procedure or subjecting the patient to radiation.

In this exemplary embodiment, the first accelerometer 120A is mounted on the medial side of the knee joint and is operative to detect vibrations generated by the interactions between the medial condyle of the femur against the medial cartilage on top of the medial portion of the tibia. Similarly, the second accelerometer 120B is mounted on the lateral side of the knee joint and is operative to detect vibrations generated by the interactions between the lateral condyle of the femur against the lateral cartilage on top of the lateral portion of the tibia. Finally, the third accelerometer 120C is mounted at the front of the knee joint, proximate the patella, and is operative to detect vibrations generated by the interactions between the femur against the patella. The resulting data output by the accelerometers 120 is wirelessly transmitted to the computer 108 via a wireless transmitter 124, such as an ultra wide band transmitter. The data from the accelerometers 120 that is wirelessly transmitted to the computer is then utilized in combination with data from the other modules to ascertain the appropriate diagnosis.

Referring back to FIG. 4, the system 100 includes a contact force module (CFM) 104 comprising a plurality of pressure sensors 130 that are utilized to detect pressure or contact forces occurring at the bottom of the foot when the knee joint is moved through a range of motion under a loaded condition. In other words, as the patient walks, jogs, runs, etc., the CFM module 104 detects pressure data at the bottom of the foot when the foot is partially or fully in contact with the ground. In exemplary form, the pressure sensors 130 are incorporated into an insole that conforms to the general shape of a patient's foot. Because humans have different sized feet, the exemplary system includes insoles that are incrementally sized to accommodate humans with different sized feet or to accommodate the shoes (or lack thereof) needed for a certain activity.

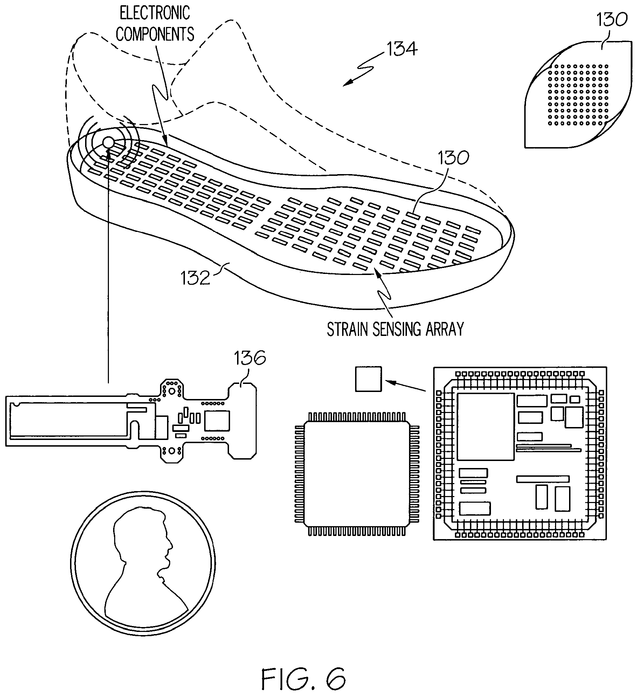

Referring to FIG. 6, the exemplary CFM 104 includes pressure sensors 130 that are arranged in a grid on the insole 132 of a shoe 134. In exemplary form, the grid comprises a series of rows and columns of pressure sensors that are exposed to the underside of a patient's foot so that contact forces applied by the foot to the shoe, by way of the insole, can be measured, as well as knowing the location where the forces were applied. As will be discussed in more detail hereafter, the location of the pressures and the relative amount of pressures provides information relevant to diagnosis of injury. For example, a patient with a limp, caused by a knee joint injury, would not apply pressure to the sole of a shoe in the same manner (amount or location) as would a patient with a healthy knee joint and a normal gait or kinematics.

Each of the capacitive sensors 130, as the name implies, includes a capacitor that works on the principle that a deformable dielectric medium separates two plates. Changes in the pressure applied to the plates cause a strain (deformation) within the dielectric medium. Thus a pressure applied to the sensor changes the spacing between the plates and changes the capacitance measured between them. The sensors 130 are arrayed across the area of pressure measurement to provide discrete capacitive data points corresponding to strains at the various locations of the array. These strains are used to find the stresses and thus the forces to calculate the output of pressure data having units of force per unit area multiplied by time (i.e., $N/m^2$ sec).

In this exemplary embodiment, the sensors 130 are arranged in a grid so that the position of each of the sensors relative to another sensor is known. This data, which includes the 2D orientation and spacing between the pressure sensors 130, is either stored on the computer 108 or stored locally with the sensors. In this exemplary embodiment, the orientation and spacing data for the sensors 130 is stored on the computer 108. The resulting data output by the sensors 130 is wirelessly transmitted to the computer 108 via a wireless transmitter 136, such as an ultra wide band transmitter. Using the orientation and spacing data for the sensors 130 stored on the computer 108, in combination with the computer receiving sensor pressure data, the computer is operative to generate data tying pressure to position, specifically the position of one pressure sensor with respect to another.

By tying force with position, the system 100 includes data reflecting precisely what pressures are exerted at what locations. In addition, the computer 108 includes an internal clock that also associates time with the pressure data generate by the pressure sensors 130. Accordingly, the system 100 not only knows how much pressure was exerted and the location where the pressure was applied, but also has time data indicating the duration of the applied pressures. Again, by tying the pressure data generated by the pressure sensors 130 to time, the pressure data can be correlated with the sound data generated by the VM module 102 using a time scale as a common scale. As a result, the system can evaluate how pressures exhibited at the bottom of the foot change as a function of time, along with how the vibrational data changes during the same time.

Referencing FIG. 4 again, the system 100 also includes a kinematics module (KM) 106 that is comprise of a plurality of submodules 140, 142, 144 that include one or a plurality of A-mode ultrasound transducers. The submodules include an ultrasound creation and positioning submodule 140, an ultrasound registration submodule 142, and an ultrasound dynamic movement submodule 144. Specifically, the submodules include A-mode ultrasound transducers that generate sound and detect the sound that bounces back, which is representative of the structure, position, and acoustical impedance of the tissue in question. Commercially available transducers for use with the exemplary embodiments include, without limitation, the Olympus immersion unfocused 3-5MHz transducer. Those skilled in the art are familiar with the operation of ultrasound transducers in general and A-mode ultrasound transducers, which generate sound pulses and operate to detect sound that bounces back within soft tissue at the interface between tissues having different acoustic impedances. The magnitude of the sound that bounces back and the time it takes for the sound to bounce back to the ultrasound transducer are utilized to determine the distance between the ultrasound transducer and the interface between the materials having different acoustic impedances.

In this exemplary embodiment, the transducers are utilized to detect the interface between bone and surrounding tissue so that the location of the bone surface can be determined. Because the operation of ultrasound transducers and A-mode ultrasound transducers are well known to those skilled in the art, a detailed discussion of the operation of ultrasound transducers in general, and A-mode ultrasound transducers specifically, has been omitted only for purposes of brevity.

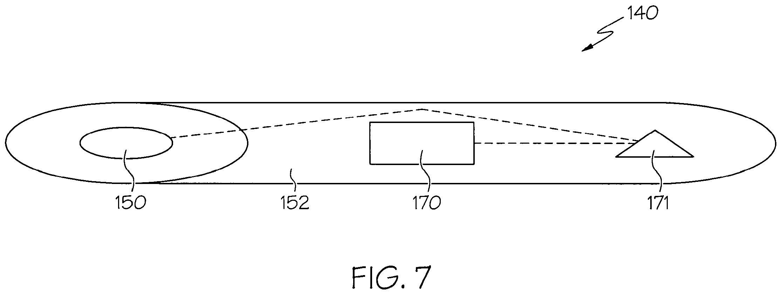

Referring to FIG. 7, the ultrasound creation and positioning submodule 140 comprises one or more A-mode ultrasound transducers 150 fixedly mounted to a wand 152 that also has mounted thereto at least one positioning device 170. In this exemplary embodiment, the ultrasound creation and positioning submodule 140 is physically separate from the ultrasound registration submodule 142 and an ultrasound dynamic movement submodule 144, which are themselves mounted to a rigid knee brace 190 (see FIG. 5). In this fashion, the ultrasound creation and positioning submodule 140 is repositionable with respect to the rigid knee brace 190 and adapted to place one or more of its A-mode ultrasound transducers 150 in contact with the patient's epidermis proximate the knee joint. It should be noted, however, that the knee brace 190 does not have to be rigid other than the linkages between certain components. Moreover, the knee should be scanned by the ultrasound wand 152 before the brace is put on.

One of the functions of the ultrasound creation and positioning submodule 140 is to generate electrical signal representative of the ultrasonic wave detected by the transducers 150 as the wand 152 is moved over the patient's epidermis proximate the knee joint. The ultrasound transducer(s) 150 receive electrical signal pulses based upon the magnitude of the ultrasonic wave that bounces back to the transducer as a result of the sound reaching the bone and bouncing back. As discussed previously, the magnitude of the electrical signal and the delay between the generation of the ultrasonic wave by the ultrasound transducer 150 until a bounce back ultrasonic wave is detected by the ultrasound transducer is indicative of the depth of the bone underneath the transducer. But this depth data alone would not be particularly useful without positioning devices 170 that provide a 3D coordinate system.

In an embodiment not forming part of the claimed invention, the positioning devices 170 of the ultrasound creation and positioning submodule 140 are fixedly mounted to the wand 152 and may comprise any of a number of devices. For example, the wand 152 may include optical devices 170 that are operative to generate, detect, or reflect pulses of light, which interacts with a corresponding detector or light generator to discern the 3D position of the wand with respect to a fixed or reference position. One such device includes a light detector operative to detect pulses of light emitted from light emitters having known positions. The light detector detects the light and sends, a representative signal to control circuitry, which also knows when the light pulses were emitted as a function of time and position. In this matter, the control circuitry is operative to determine the position of the wand in a 3D coordinate system. Because the A-mode ultrasound transducer(s) 150 and optical devices 170 are fixedly mounted to the wand 152, the position of the ultrasound transducer(s) 150 with respect to the position of the optical devices 170 is known. Similarly, because the ultrasound transducers 150 are generating signals representative of a straight line distance from the transducer to the surface of the bone, and the position of the transducer(s) 150 with respect to the optical devices 170 is known, the position of the bone with respect to the optical devices 170 can be easily calculated. In other words, as the wand 152 is repositioned, the optical devices 170 generate data reflecting that the relative position of the optical devices has changed in the 3D coordinate system. This change in 3D position of the optical devices 170 can be easily correlated to the position of the bone in three dimensions because the position of the bone relative to the ultrasound transducer is known, as is the position of the optical devices with respect to the ultrasound transducers. Accordingly, the optical devices 3D position data is used in combination with the fixed position data (distance data for the position of the ultrasound transducer(s) with respect to the optical devices) for the ultrasound transducers 150 in combination with the distance data generated responsive to the signals received from the ultrasound transducers to generate composite data that is used to create a plurality of 3D points representing a plurality of distinct points on the surface of the bone. As will be discussed in more detail below, these 3D points are utilized in conjunction with a default bone model to generate a virtual, 3D representation of the patient's bone.

The positioning devices 170 comprise one or more inertial measurement units (IMUs). IMUs are known to those skilled in the art and include accelerometers, gyroscopes, and magnetometers that work together to determine the position of the IMUs in a 3D coordinate system. Because the A-mode ultrasound transducer(s) 150 and IMUs 170 are fixedly mounted to the wand 152, the position of the ultrasound transducers 150 with respect to the position of the IMUs 170 is known. Similarly, because the ultrasound transducers 150 are generating signals representative of a straight line distance from the transducer to the surface of the bone, and the position of the transducer(s) 150 with respect to the IMUs 170 is known, the position of the bone with respect to the IMUs 170 can be easily calculated. In other words, as the wand 152 is repositioned, the IMUs 170 generate data indicating that the relative position of the IMUs has changed in the 3D coordinate system. This change in 3D position of the IMUs 170 can be easily correlated to the position of the bone in three dimensions because the position of the bone relative to the ultrasound transducer is known, as is the position of the IMUs with respect to the ultrasound transducers. Accordingly, the IMU 3D position data is used in combination with the fixed position data (distance data for the position of the ultrasound transducer(s) with respect to the IMUs) for the ultrasound transducers 150 in combination with the distance data generated responsive to the signals received from the ultrasound transducers to generate composite data that is used to create a plurality of 3D points representing a plurality of distinct points on the surface of the bone. As will be discussed in more detail below, these 3D points are utilized in conjunction with a default bone model to generate a virtual, 3D representation of the patient's bone.

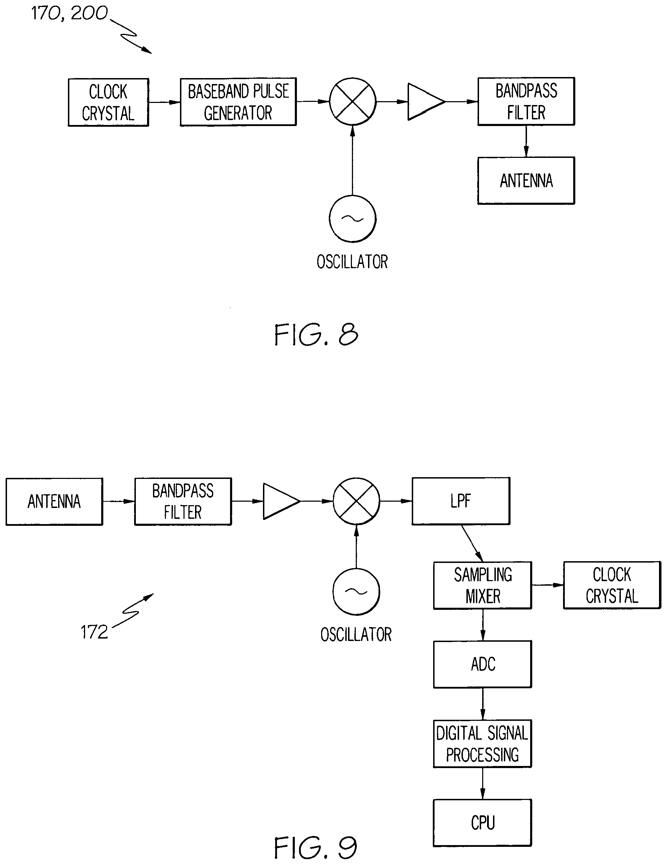

Referring to FIGS. 8-10, the positioning devices 170 may alternatively comprise one or more ultra wide band (UWB) transmitters. UWB transmitters are known to those skilled in the art, but the use of UWB transmitters and receivers for millimeter-accuracy 3D positioning is novel. One or more UWB transmitters 170 is fixedly mounted to the wand 152 and is operative to transmit sequential UWB signals to a three or more UWB receivers (having known positions in a 3D coordinate system). The UWB positioning system is comprised of active tags or transmitters 170 that are tracked by the UWB receivers 172. The system architecture of the UWB transmitter 170 is shown in FIG. 8, where a low noise system clock (clock crystal) triggers a baseband UWB pulse generator (for instance a step recovery diode (SRD) pulse generator). The baseband pulse is upconverted by a local oscillator (LO) via a double balanced wideband mixer. The upconverted signal is amplified and filtered. Finally, the signal is transmitted via an omnidirectional antenna. The UWB signal travels through an indoor channel where significant multipath and pathloss effects cause noticeable signal degradation.

Referencing FIG. 9, the UWB receiver 172 architecture is shown, where the signal is received via a directional UWB antenna and is filtered, amplified, downconverted, and low-pass filtered. Next, a sub-sampling mixer triggered by a second low noise system clock is used to time extend the pulse by 1000-100,000x. This effectively reduces the bandwidth of the UWB pulse and allows sampling by a conventional analog-to-digital converter (ADC).

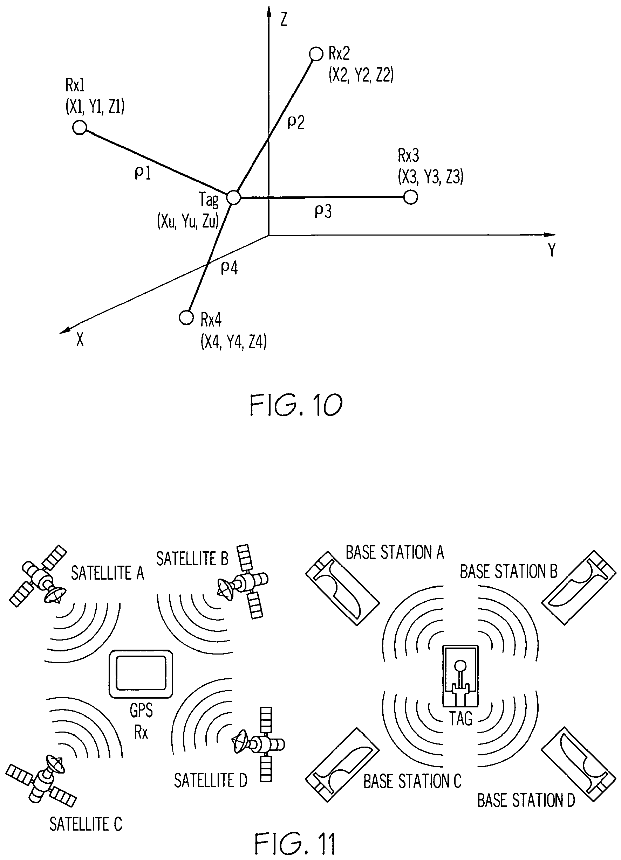

Each UWB transmitter 170 is in communication with the computer 108, as are the UWB receivers 172. Accordingly, the computer 108 is aware each time the UWB transmitter transmits a UWB signal, as well as the time that the UWB transmitter transmits the UWB signal. Similarly, the computer 108 is aware of the position of each of the UWB receivers in a 3D coordinate system, as well as the time during which each of the UWB receivers receives the UWB signal from the UWB transmitter. By knowing the position of each UWB receiver, the time when each UWB receiver receives the UWB signal from the UWB transmitter, and the time that the UWB transmitter transmitted the UWB signal, the computer 108 uses custom digital signal processing algorithms to accurately locate the leading-edge of the received UWB pulse to within sub-sample resolution. The final time-difference-of-arrival (TDOA) calculation (see FIG. 10) as well as additional filtering and averaging of data is also carried out by the computer 108.

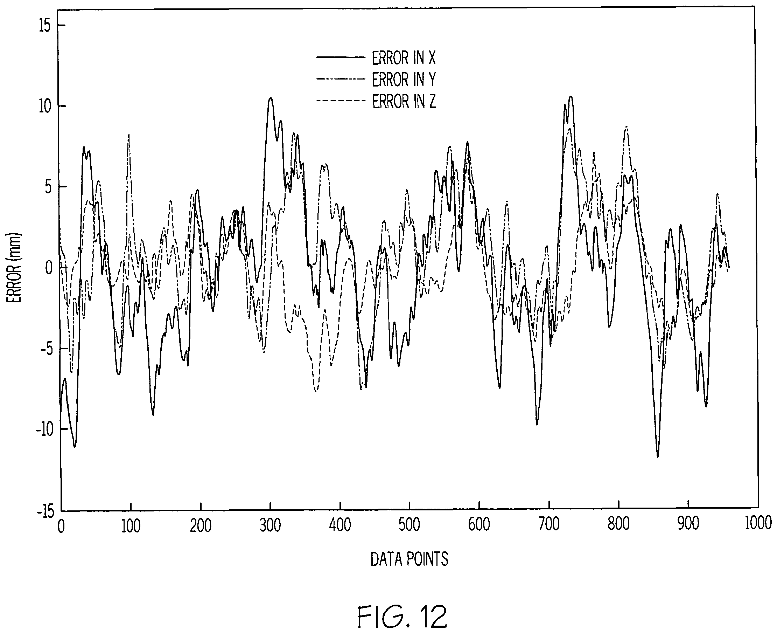

At least four base stations (receivers) 172 are needed to localize the 3D position of the UWB transmitter 170. The geometry of the receivers 172 has important ramifications on the achievable 3D accuracy through what is known as geometric position dilution of precision (PDOP). A combination of novel filtering techniques, high sample rates, robustness to multipath interference, accurate digital ranging algorithms, low phase noise local oscillators, and high integrity microwave hardware are needed to achieve millimeter range accuracy (e.g. 5-7 mm 3D realtime). The analogy of the UWB positioning system to a GPS system is shown in FIG. 11. Finally, FIG 12 shows actual experimental errors in x,y,z coordinates for detecting the 3D position of the UWB transmitter 170 in real-time over 1000 samples while the transmitter is moving freely within the designated view volume.

Because the A-mode ultrasound transducer(s) 150 and UWB transmitter(s) 170 are fixedly mounted to the wand 152, the position of the ultrasound transducer(s) 150 with respect to the position of the UWB transmitter(s) 170 is known. Similarly, because the ultrasound transducers 150 are generating signals representative of a straight line distance from the transducer to the surface of the bone, and the position of the transducer(s) 150 with respect to the UWB transmitter(s) 170 is known, the position of the bone with respect to the UWB transmitter(s) 170 can be easily calculated. In other words, as the wand 152 is repositioned, the UWB transmitter(s) 170 transmit UWB signals, which are correspondingly received by the UWB receivers. This information is processed by the computer 108 in order to discern whether the relative position of the UWB transmitter(s) has changed in the 3D coordinate system, as well as the extent of such a change. This change in 3D position of the UWB transmitter(s) 170 can be easily correlated to the position of the bone in three dimensions because the position of the bone relative to the ultrasound transducer is known, as is the position of the UWB transmitter(s) with respect to the ultrasound transducers. Accordingly, the UWB transmitter(s) 3D position data is used in combination with the fixed position data (distance data for the position of the ultrasound transducer(s) with respect to the UWB transmitter(s)) for the ultrasound transducers 150 in combination with the distance data generated responsive to the signals received from the ultrasound transducers to generate composite data that is used to create a plurality of 3D points representing a plurality of distinct points on the surface of the bone. As will be discussed in more detail below, these 3D points are utilized in conjunction with a default bone model to generate a virtual, 3D representation of the patient's bone.

Regardless of the positioning devices 170 utilized with the ultrasound creation and positioning submodule 140, the wand 152 is repositioned over the skin of a patient, proximate the knee joint, while the knee joint is bent in order to individually, and successively map (creation of 3D points corresponding to points on the surface of the patient's bone) the three bones of the knee joint (distal femur, proximal tibia, and patella). As the wand 152 is repositioned, the data from the transducer(s) 150 is transmitted to a wireless transmitter 171 mounted to the wand 152. When the wireless transmitter receives the data from the transducer(s) 150, the transmitter transmits the data via a wireless link to the computer 108.

In order to power the devices on-board the wand 152, an internal power supply (not shown) is provided. In exemplary form, the internal power supply comprises one or more rechargeable batteries.

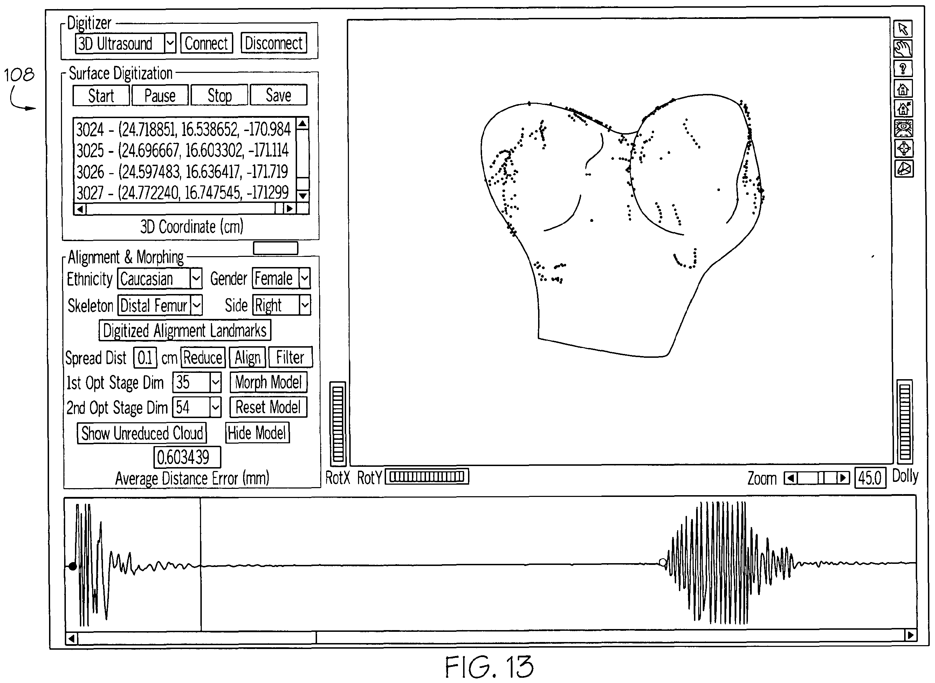

Referring to FIG. 13, before patient data is taken, the computer 108 software requests a series of inputs to adapt the system to equipment specific devices and the particular bone being modeled. For example, a dropdown menu on the user interface allows the user to input precisely what type of digitizer will be utilized, which may include, without limitation, ultrasound. After the type of digitizer is selected, the user may actuate buttons to connect to or disconnect from the digitizer. Before, during, or after the ultrasound transducer data is acquired, the software provides various dropdown menus allowing the software to load a bone model that roughly is the same shape as the patient's bone.

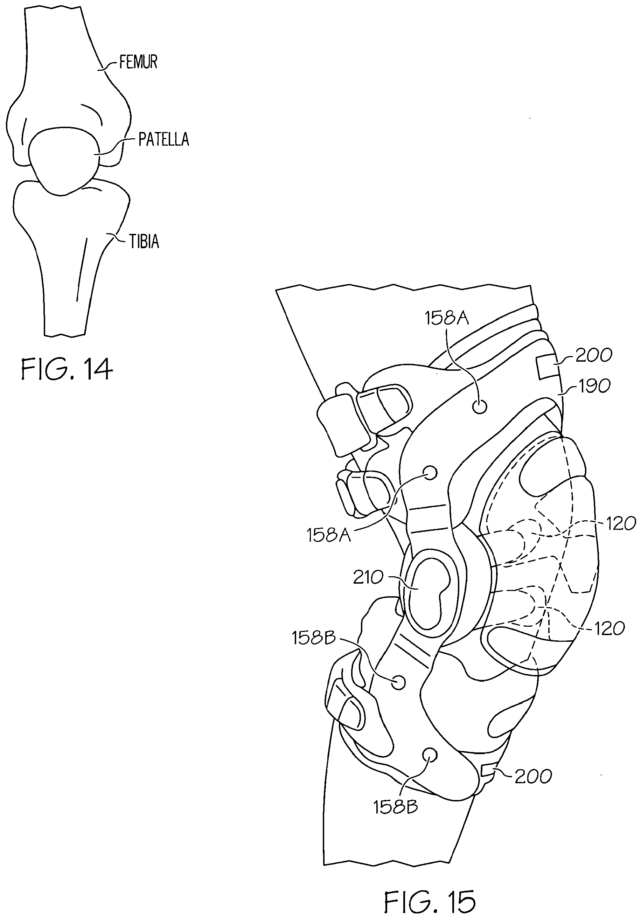

After the computer 108 receives the ultrasound transducer 150 data, the computer 108 includes software that interprets the A-mode ultrasound transducer data and is operative to construct a 3D map having discrete 3D points that correspond to points on the surface of the bone in question. Consequently, the wand 152 (see FIG. 7) is repositioned over the bones (distal femur, patella, proximal tibia) for approximately 30 second so that the discrete points typify the topography of the bone. Consequently, repositioning the wand 152 over the bone in question for a longer duration results in more 3D points being generated by the computer 108, which consequently helps ensure a more accurate patient-specific bone model, such as that shown in FIG. 14. A partial range of motion of the knee joint while repositioning the wand 152 over the joint can help the wand view new portions of the bone in question for new 3D points that may have been obscured by other bones in another range of motion position.

After each of the bones has been mapped, the computer 108 then uses a default bone model as a starting point to construction of the ultimate patient specific, virtual bone model. The default bone model may be a generalized average, as the morphing algorithms use statistical knowledge of a wide database population of bones for a very accurate model. However, for expedited computation, a more generalized default bone model may be selected based upon the patient's gender, race, height, age, for example, as a starting point. For example, in the case of generating a patient-specific model of the femur where the patient is a 53 year old, Caucasian male, who is 183cm (six feet) tall, a default femoral bone model is selected based upon the classification of Caucasian males having an age between 50-60, and a height between 178cm and 188cm (5'10" and 6'2"). In this manner, selection of the appropriate default bone model more quickly achieves an accurate patient specific, virtual bone model because the iterations between the patient's actual bone (typified by the 3D map of bone points) and the default bone model are reduced. After the appropriate default bone model is selected, the computer superimposes the 3D map of actual bone points onto the default bone model and thereafter carries out a deformation process so that the bone model exhibits the actual bone points detected during the wanding. The deformation process also makes use of statistical knowledge of the bone shape based upon reference bones of a wide population. After the deformation process is carried out, the resulting bone model is a patient-specific, virtual 3D model of the patient's actual bone. The foregoing process is carried out for each of the three bones of the knee joint to create patient-specific, virtual 3D models of the patient's proximal tibia, distal femur, and patella.

Referring back to FIGS. 4, 5, and 15 the ultrasound registration submodule 142 and the ultrasound dynamic movement submodule 144 are mounted to a knee brace 190. The data output from these submodules 142, 144 is utilized to generate dynamic 3D models of the patient's own bones through a range of motion. In other words, a visual terminal associated with the computer 108 can display the patient's virtual own bone models moving in 3D that match the same movement of the patient's own bones. As will be discussed later, this dynamic 3D model taken through a range of motion is part of what the neural network analyzed to determine if an injury exists and the extent of the injury.

In exemplary form, the ultrasound registration submodule 142 comprises a plurality of A-mode ultrasound transducers 158 fixedly mounted to the knee brace 190. Specifically, in the context of a knee joint, there are at least two A-mode ultrasound transducers 158 (i.e., a transducer group) fixedly mounted to the knee brace 190 for tracking of the tibia and femur. In other words, the knee brace 190 includes at least six ultrasound transducers in order to track the two primary bones of the knee joint. Each transducer group 158 includes a rigid mechanical connection linking the transducers 158, the positioning devices 200, and a connection the knee brace 190 which may or may not be rigid. Each transducer group 158 includes a rigid mechanical connection linking the transducers 158, the positioning devices 200, and the knee brace 190. In this manner, the relative positions of the transducers with respect to one another do not change. In exemplary form, a first transducer group 158A at least partially circumscribes a distal portion of the femur, while a second transducer group 158B at least partially circumscribes a proximal portion of the tibia, while an optional third transducer group (not shown) overlies the patella if patella kinematics are to be tracked. The ultrasound registration submodule 142 is accordingly operative to provide a plurality of static reference points for each bone as the bone is moved through a range of motion.

Referring back to FIG. 4, the ultrasound dynamic movement submodule 144 comprises a plurality of positioning devices 200 that are operative to feed information to the computer 108 regarding the 3D position of each transducer group 158A, 158B of the ultrasound registration submodule 142. In a purely exemplary form, though the invention of claim 1 uses inertial measurement, the positioning devices 200 may comprise light detectors operative to detect pulses of light emitted from light emitters having known positions. The light detectors 200 detect the light and send representative signals to control circuitry associated with the knee brace 190. The knee brace 190 transmits this information to the computer 108 (see FIG. 4), which also knows when the light pulses were emitted as a function of time and position. In this manner, the computer can determine the position of the transducers 158 in a 3D coordinate system. Because the A-mode ultrasound transducer(s) 158 and optical devices 200 are fixedly mounted to the knee brace 190, the position of the ultrasound transducer(s) 158 with respect to the position of the optical devices 200 is known. Similarly, because the ultrasound transducers 158 are generating signals representative of a straight line distance from the transducer to a surface of the bone, and the position of the transducer(s) 158 with respect to the optical devices 200 is known, the position of the bone with respect to the optical devices 200 can be easily calculated. **In** other words, as the knee is repositioned, and correspondingly so too is the knee brace 190, the optical devices 200 generate data reflecting that the relative position of the optical devices has changed in the 3D coordinate system. This change in 3D position of the optical devices 200 can be easily correlated to the position of the bone in question (femur, patella, or tibia) in three dimensions because the position of the bone relative to the ultrasound transducer groups 158A, 158B is known, as is the position of the optical devices with respect to the ultrasound transducer groups. Accordingly, the optical devices generate data that is used in combination with the fixed position data (distance data for the position of the ultrasound transducer(s) with respect to the optical devices) for the ultrasound transducers 158 in combination with the distance data generated responsive to the signals received from the ultrasound transducers to generate composite data that is used to create dynamic movement map of the bone in question. By way of example, because the transducers 158 do not move with respect to the positioning devices 200, any movement of the transducers 158 in space means that the positioning devices 200 have moved in 3D space, and by continuing to track the distance data provided by each transducer 158, the movement of the bone in question can be correspondingly tracked.

Alternatively, the positioning devices 200 may comprise one or more inertial measurement units (IMUs). IMUs are known to those skilled in the art and include a combination of accelerometers, gyroscopes, and magnetometers that work together to determine the position of the IMUs in a 3D coordinate system. IMUs are known to those skilled in the art and include accelerometers, gyroscopes, and magnetometers that work together to determine the position of the IMUs in a 3D coordinate system. Because the A-mode ultrasound transducer(s) 158 are fixedly mounted to the IMUs 200, the position of the ultrasound transducer(s) 158 with respect to the position of the IMUs 200 is known. Similarly, because the ultrasound transducers 158 are generating signals representative of a straight line distance from the transducer to the surface of the bone, and the position of the transducer(s) 158 with respect to the IMUs 200 is known, the position of the bone with respect to the IMUs 200 can be easily calculated. In other words, as the brace 190 is repositioned, the IMUs 200 generate data indicating that the relative position of the IMUs has changed in the 3D coordinate system. This change in 3D position of the IMUs 200 can be easily correlated to the position of the bone in 3D because the position of the bone relative to the ultrasound transducer is known, as is the position of the IMUs with respect to the ultrasound transducers. By way of example, because the transducers 158 do not move with respect to the brace 190, any movement of the transducers 158 in space means that the brace has moved in 3D space, and by continuing to track the distance data provided by each transducer 158, the movement of the bone in question can be correspondingly tracked. Such an IMU 200 allows relative tracking of the bone movements and requires a static registration between the multiple IMU units with an initial known position (such as standing). Thus the IMU enables measurement of the relative motion between different bones via their corresponding ultrasound transducer array data 158 and IMUs 200 data. The IMU 200 may be used alone or in conjunction with another positioning device 200 such as those described in paragraphs 0059 and 0061. The IMU 200 may also be used in conjunction with another positioning devices 200 such as those described in paragraphs 0060 and 0062. In this scenario, the IMUs 200 position is updated at a certain interval with the absolute position from the other positioning system 200 as a reference to minimize errors, so the two positioning systems 200 act together as one positioning system 200.

Referring to FIGS. 8-10, the positioning devices 200 may alternatively comprise one or more ultra wide band (UWB) transmitters. UWB transmitters are known to those skilled in the art, but the use of UWB transmitters and receivers for 3D positioning is believed to be novel. One or more UWB transmitters 200 is fixedly mounted to the brace 190 and is operative to transmit sequential UWB signals to a three or more UWB receivers (having known positions in a 3D coordinate system). Each UWB transmitter 200 is in communication with the computer 108, as are a plurality of UWB receivers 202. Accordingly, the computer 108 is aware each time the UWB transmitter transmits a UWB signal, as well as the time that the UWB transmitter transmits the UWB signal. Similarly, the computer 108 is aware of the position of each of the UWB receivers in a 3D coordinate system, as well as the time during which each of the UWB receivers receives the UWB signal from the UWB transmitter. By knowing the position of each UWB receiver, the time when each UWB receiver receives the UWB signal from the UWB transmitter, and the time that the UWB transmitter transmitted the UWB signal, the computer 108 uses custom digital signal processing algorithms to accurately locate the leading-edge of the received UWB pulse to within sub-sample resolution. The final time-difference-of-arrival (TDOA) calculation (see FIG. 10) as well as additional filtering and averaging of data is also carried out by the computer 108. Again, because the transducers 158 do not move with respect to the brace 190, any movement of the transducers 158 in space means that the brace has moved in 3D space. Yet the movement of the brace is tracked using the computer 108 in combination with the UWB transmitter and UWB receivers. Similarly, because the fixed orientation of the UWB transmitter and the ultrasound transducers 158, changes in position in a 3D coordinate system of the UWB transmitter 200 can correspondingly be used to track movement of the bone in question.

In order to communicate information from the submodules 142, 144 to the computer 108, the brace 190 includes an ultra wide band (UWB) transmitter 210 in communication with the ultrasound transducers 158 to facilitate wireless communication of data to the computer 108. It should be noted that if UWB transmitters are utilized as the positioning devices 200, a dedicated UWB transmitter is unnecessary as the UWB transmitters 200 could function to send ultrasound sensor data directly to the computer 108 over a wireless link.

It should be understood that the use of wireless transmitters and a field programmable gate array design enables the computations to be carried out on a realtime basis, with final processing and display carried out on the computer 108. It should be understood that the use of wireless transmitters and a computer 108 incorporating a field programmable gate array design enables the computations to be carried out on a real-time basis. For example, as the wand 152 is utilized to go across the epidermis covering the knee joint, the ultrasound transducer data is immediately transmitted to the computer, which in real time calculates the position of the bone in a 3D coordinate system and likewise displays the 3D points on a visual terminal again in real time. Similarly, when the knee brace 190 is utilized, the ultrasound transducer data and positioning device data is transmitted to the computer and evaluated in real time to provide motion to the static, 3D patient-specific bone models previously generated. Again, when the computer includes a visual terminal, the dynamic motion imparted to the 3D patient-specific bone models tracks in real time the actual motion of the patient's bones.

As discussed above, an exemplary knee brace 190 includes a plurality of A-mode ultrasound transducers 158 for transcutaneous detection of the bone's surface and positioning devices 200 to track the motion of the ultrasound transducers 158, which in turn, track motion of the knee joint bones. The brace 190 is wirelessly connected to a computer 108 operative to perform computations and visualization in real-time showing movements of the patient-specific 3D bone models paralleling movements of the patient's actual knee joint in a time synchronized manner. The exemplary brace 190 includes a rigid or semi-rigid body having a plurality positioning devices 200 attached thereto. An even further alternate positioning device 200 includes a plurality of accelerometers in this case at least four accelerometers. The homogenous transformation between an accelerometer's reference coordinate frame and the world coordinate frame is calculated using the positions of the four accelerometers:

(1)

$$v(n+1) = v(n) + a(n)dt$$

(2)

$$s(n+1) = s(n) + v(n)dt - 0.5a(n)dt2$$

where s(n+1) is position at the current state, s(n) is the position from previous state, v(n+1) is instantaneous velocity of the current state, v(n) is the velocity from previous state, a(n) is the acceleration from the accelerometer and dt is the sampling time interval. The previous equations describe the dynamic motion and positioning of a point in 3D Euclidean space. Additional information is needed to describe a 3D body orientation and motion.

The orientation of the transducer can be described by using a gravity based accelerometer (example: ADXL-330, analog device) by extracting the tilting information from each pair of orthogonal axis The acceleration output on x ,y, or z due to gravity is equal to the following: Ai = (Vout$_x$ - Voft) / S, where Ai is the acceleration at the x, y, or z axis, Vout$_x$ is the voltage output from the x, y, or z axis, Voff is the offset voltage, and S is the sensitivity of the accelerometer the yaw, pitch and roll can be calculated as shown in the following:

$$\rho = arctan\left(\frac{Ax}{\sqrt{A_y^2 + A_z^2}}\right)$$

$$\emptyset = arctan\left(\frac{Ay}{\sqrt{A_x^2 + A_z^2}}\right)$$

$$\theta = arctan\left(\frac{\sqrt{A_y^2 + A_z^2}}{Az}\right)$$

where pitch is $\rho$, which is the x-axis relative to the ground, roll is Ø, which is the y-axis relative to the ground, and roll is $\theta$, which is the z-axis relative to the ground. Since the accelerometer is gravity based, the orientation does not require information from the previous state once the sensor is calibrated. The static calibration requires the resultant sum of accelerations from the three axis equal to1g (the nominal acceleration due to gravity at the Earth's surface at sea level, defined to be precisely 9.80665 m/s$^2$ (approximately, 32.174 ft/s$^2$)). Alternatively, an orientation sensor that provides yaw, pitch, and roll information of the bodily tissue in question are also commercially available (e.g., IDG-300, available from Invensense). The orientation of the transducer can then be resolved by using direction cosine matrix transformation:

$$\begin{bmatrix} X_2 \\ Y_2 \\ Z_2 \end{bmatrix} = \begin{bmatrix} C\theta C\emptyset & C\theta S\emptyset S\rho - S\theta C\rho & C\theta S\emptyset C\rho - S\theta S\rho \\ S\theta C\emptyset & S\theta S\emptyset S\rho - C\theta C\rho & S\theta S\emptyset C\rho - C\theta S\rho \\ -S\emptyset & C\emptyset S\rho & C\theta C\rho \end{bmatrix} \begin{bmatrix} X_1 \\ Y_1 \\ Z_1 \end{bmatrix}$$

where C represents cosine and S represents sine.

The interpretation of the vibration and kinematic data is a complicated task involving an in-depth understanding of data acquisition, training data sets and signal analysis, as well as the mechanical system characteristics. Vibrations generated through the implant components, bones, and/or soft tissues interaction result from a forced vibration induced by driving force leading to a dynamic response. The driving force can be associated with the impact following knee ligament instability, bone properties, and conditions. A normal, intact knee will have a distinct pattern of motion, coupled with distinct vibrational characteristics. Once degeneration or damage occurs to the knee joint, both the kinematic patterns and vibrational characteristics become altered. This altering, for each type of injury or degeneration, leads to distinct changes that can be captured using both kinematic and vibration determination.

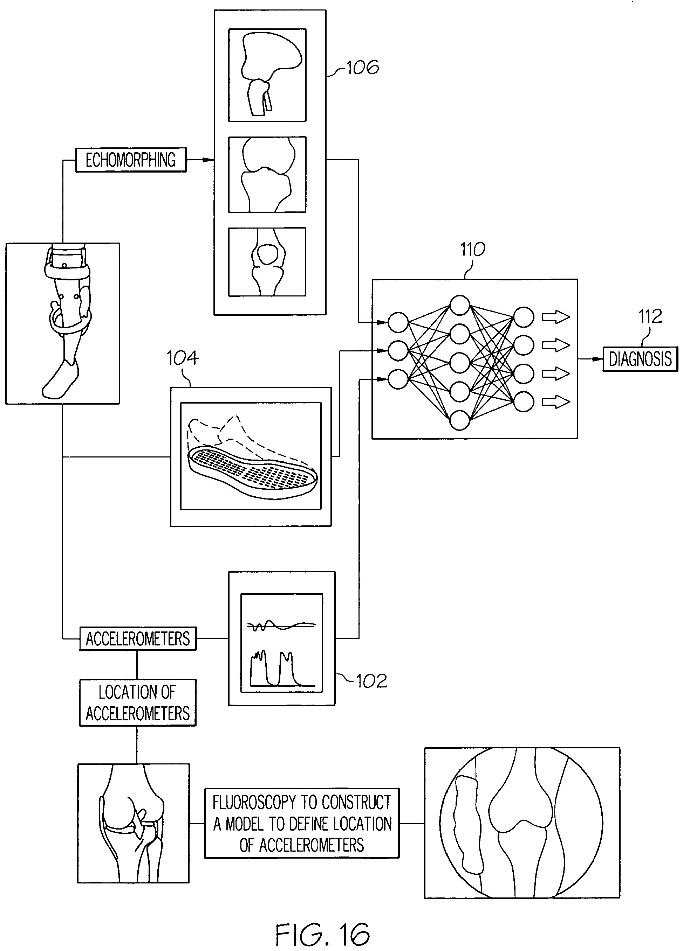

Referencing FIGS. 16-19, the exemplary diagnostic system 100 includes an intelligent diagnosis module 110 operative to diagnose ligament, other soft tissue, and bone injuries. From previous studies, normal and anterior cruciate ligament deficient (ACLD) knee subjects exhibit a similar pattern of posterior femoral translation during progressive knee flexion, but the subjects exhibit different axial rotation patterns of 30 degrees of knee flexion. Accordingly, the diagnosis module 110 includes three stages: (1) a first stage that involves data gathering and analysis; (2) detection of an injury by sending the data to a neural network; and (3) another stage of a neural network that classifies or determines the severity of any injury that is detected.

This first stage includes acquisition of kinematic feature vectors using multiple physiological measurements taken from the patient while the patient moves the joint in question through a range of motion. Exemplary measurements include, without limitation, medial condyle anteroposterior motion (MAP) and lateral condyle anteroposterior (LAP), with the latter pertaining to the anterior-posterior A/P distance of the medial and lateral condyle points relative to the tibia geometric center. Other exemplary measurements include LSI (distance between the lateral femoral condyle and the lateral tibial plateau) and MSI (distance between the medial femoral condyle and the medial tibial plateau) which are S/I (superior/inferior) distance of the lateral and medial condyle points to the tibial plane. Further exemplary measurements include condyle separation, which is the horizontal (x-y plane) distance between the two minimum condyle points to the tibia. Feature vectors also include the femoral position with respect to the tibia which is defined by three Euler angles and three translation components in addition to the vibration signal, and force data.

Referring to FIG. 16, the motion features vectors, extracted from the kinematic and vibration analyses, are output to a multilayer back propagation neural network for determining the injured ligament.

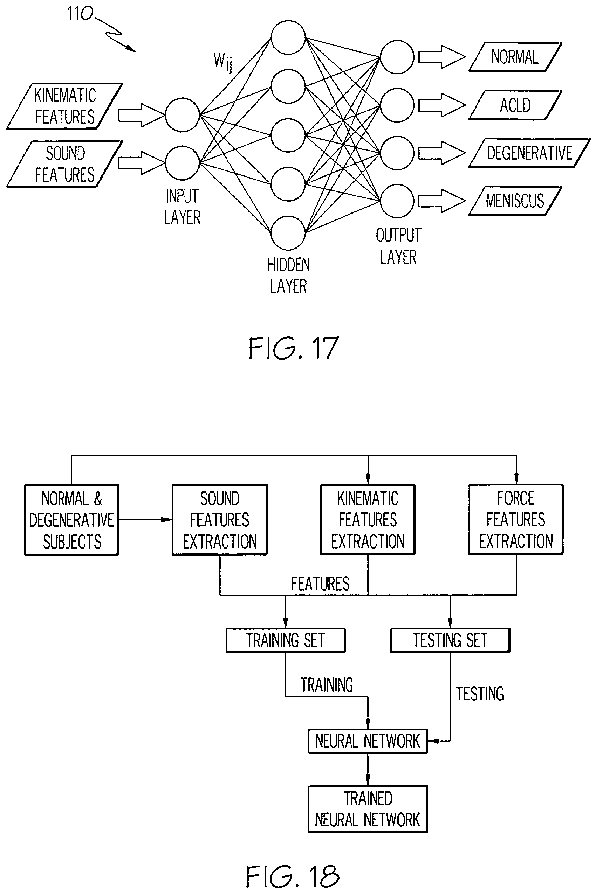

Referencing FIG. 17, an exemplary neural network classifier has multiple binary outputs. Each output is either a one or zero, with one corresponding to yes and zero corresponding to no. In this exemplary neural network classifier, each output represents the response of the neural network to a particular injury type; for example one output will represent the response for anterior cruciate ligament deficiency (ACLD), its state will be one if an ACL injury is detected, and zero otherwise. Obviously, the neural network may be significantly more sophisticated or less sophisticated, depending upon the underlying model of the joint in question.

Referring to FIG. 18, construction of the exemplary neural network includes formulating a supervised classifier using a training set of the kinematic and vibration data corresponding to normal and injured knee joint. The NN is trained with a set of vectors. Each vector consists of data (kinematics, vibrations and forces) collected from one joint. Fluoroscopy data can be used to calculate the kinematics. Once the NN is trained, it can be used to classify new cases and categorize the injury type using these kinematics, vibration and forces data. Those skilled in the art will readily understand that the types and classifications desired to be accommodated by the neural network necessarily include training the neural network on these very types and classifications. Exemplary types and classifications of injuries to a mammalian knee joint include, without limitation, osteoarthritic conditions, soft tissue damage, and abnormal growths. Likewise, the neural network also needs to be trained as to indicators of normal knee function. In this manner, once the neural network is trained, it has the capability to differentiate between and output diagnosis data concerning normal and abnormal knee conditions.

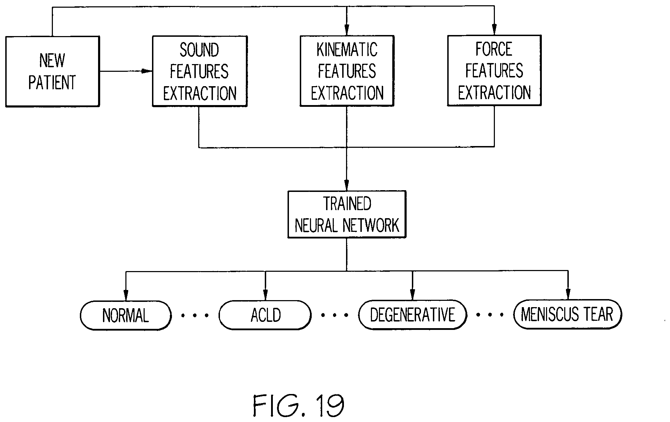

Referencing FIG. 19, the vibration, kinematics, forces, and other features of a person's knee joint are compiled and fed to the trained neural network. The trained neural network then diagnoses the condition of the patient's knee joint, identifying any degeneration by type and severity.

Exemplary embodiments may be adapted to collect data outside of a clinical setting. For example, an exemplary embodiment may be worn by a patient for an extended period of time while performing normal activities. For example, a patient may wear vibration sensors and/or a kinematics tracking brace during activities that are not reproducible in the office (for example, weight lifting, racquet ball etc.) that elicit the pain or symptom. In some embodiments, the patient may turn the device on immediately prior to the activity and/or the patient may mark the event when it occurs. This enables analysis of the data just a few seconds before the marked time to see what abnormal sounds or joint kinematic were occurring. Data may be stored on a portable hard drive (or any other portable storage device) and then may be downloaded to exemplary systems for analysis. The data can be transmitted and stored in a computer wirelessly. It can also be stored with a miniature memory drive if field data is desired. If the occurrence of the pain was more random, exemplary devices allow continuous gathering of data. In embodiments, the patient may mark the event. Devices capable of continuous monitoring may require a larger data storage capacity.

It should also be noted that electromagnetic tracking could be used as one of the positioning device 170, 200 alternatives.

It should further be noted that EMG electrodes may also be utilized as a data input for the computer 108 and neural network 110. In this fashion, one or more EMG electrodes are mounted to the surface of the skin proximate the muscles adjacent the knee joint to monitor the electrical signal transmitted to the muscles in order to provide relevant data of a muscle injury or disorder.

It is also understood that while the exemplary embodiments have been described herein with respect to a knee joint, those skilled in the art will readily understand that the aforementioned embodiments may be easily adapted to other joints of a mammalian animal. For example, embodiments may be adapted for use on hips, ankles, toes, spines, shoulders, elbows, wrists, fingers, and temporomandibular joints.

### Claims

1. A method of evaluating a physiological condition of bodily tissue, the method comprising:

   repositioning one or more A-mode ultrasound transducers (150) over a patient's epidermis to generate signals representative of a straight line distance from the one or more ultrasound transducers (150) to a bone surface of the patient;
   tracking the repositioning of the one or more ultrasound transducers in three dimensions using at least one inertial measurement unit (170) to generate 3D position data of a bone;
   correlating the signals representative of the straight line distance from the one or more ultrasound transducers (150) to the bone surface of the patient and the change in 3D position data to generate a 3D map having a plurality of 3D points corresponding to points on the bone surface;
   superimposing the 3D map onto a default bone model and carrying out a deformation process resulting in a patient-specific, virtual 3D model of the bone;
   visually displaying the virtual 3D model of the bone.

2. The method of claim 1, wherein the default bone model comprises a generalized bone model created from statistical knowledge of a wide database population of bones.

3. The method of claim 2, wherein the generalized bone model is selected based upon at least one of the patient's gender, race, height, and age.

4. The method of any one of claims 1 to 3, wherein the joint is moved through a range of motion while repositioning the one or more ultrasound transducers (150) over the patient's epidermis to generate the signals representative of a straight line distance from the one or more ultrasound transducers (150) to of a bone surface of the patient.

5. The method of any preceding claim, wherein:

   the bone comprises a first bone of a joint;
   the method is repeated for a second bone of the joint; and
   concurrently visually displaying the virtual 3D models of the first and second bones moving in 3D on a visual terminal.

6. The method of any preceding claim, wherein the at least one inertial measurement unit (170) includes a plurality of accelerometer, gyroscopes, and magnetometers.

7. The method of any preceding claim, wherein repositioning the one or more ultrasound transducers (150) over the patient's epidermis includes repositioning a wand over the patient's epidermis, where the one or more ultrasound transducers are fixedly mounted to the wand, and where the at least one inertial measurement unit (170) is fixedly mounted to the wand.

**Patentansprüche**

1. Verfahren zum Bewerten eines physiologischen Zustands von Körpergewebe, wobei das Verfahren Folgendes umfasst:

Neupositionieren eines oder mehrerer A-Modus-Ultraschallwandler (150) über der Epidermis eines Patienten, um Signale zu erzeugen, die eine geradlinige Entfernung von dem einen oder den mehreren Ultraschallwandlern (150) zu einer Knochenoberfläche des Patienten darstellen;
Verfolgen des Neupositionierens des einen oder der mehreren Ultraschallwandler in drei Dimensionen unter Verwendung mindestens einer Trägheitsmesseinheit (170), um 3D-Positionsdaten eines Knochens zu erzeugen;
Korrelieren der Signale, die die geradlinige Entfernung von dem einen oder den mehreren Ultraschallwandlern (150) zu der Knochenoberfläche des Patienten darstellen, und der Änderung der 3D-Positionsdaten, um eine 3D-Karte zu erzeugen, die eine Vielzahl von 3D-Punkten aufweist, die Punkten auf der Knochenoberfläche entsprechen;
Überlagern der 3D-Karte auf ein Standardknochenmodell und Durchführen eines Verformungsvorgangs, der zu einem patientenspezifischen virtuellen 3D-Modell des Knochens führt;
visuelles Anzeigen des virtuellen 3D-Modells des Knochens.

2. Verfahren nach Anspruch 1, wobei das Standardknochenmodell ein generalisiertes Knochenmodell umfasst, das aus statistischen Kenntnissen einer breiten Datenbankpopulation von Knochen erstellt wurde.

3. Verfahren nach Anspruch 2, wobei das generalisierte Knochenmodell basierend auf mindestens einem von Geschlecht, Ethnie, Größe und Alter des Patienten ausgewählt wird.

4. Verfahren nach einem der Ansprüche 1 bis 3, wobei das Gelenk durch einen Bewegungsbereich bewegt wird, während der eine oder die mehreren Ultraschallwandler (150) über der Epidermis des Patienten neu positioniert werden, um die Signale zu erzeugen, die eine geradlinige Entfernung von dem einen oder den mehreren Ultraschallwandlern (150) zu einer Knochenoberfläche des Patienten darstellen.

5. Verfahren nach einem vorhergehenden Anspruch, wobei:

der Knochen einen ersten Knochen eines Gelenks umfasst;
das Verfahren für einen zweiten Knochen des Gelenks wiederholt wird; und
gleichzeitiges visuelles Anzeigen der virtuellen 3D-Modelle des ersten und des zweiten Knochens, die sich in 3D bewegen, auf einem visuellen Endgerät.

6. Verfahren nach einem vorhergehenden Anspruch, wobei die mindestens eine Trägheitsmesseinheit (170) eine Vielzahl von Beschleunigungsmesser, Gyroskopen und Magnetometern enthält.

7. Verfahren nach einem vorhergehenden Anspruch, wobei das Neupositionieren des einen oder der mehreren Ultraschallwandler (150) über der Epidermis des Patienten Neupositionieren eines Stabes über der Epidermis des Patienten enthält, wobei der eine oder die mehreren Ultraschallwandler fest an dem Stab montiert sind und wobei die mindestens eine Trägheitsmesseinheit (170) fest an dem Stab montiert ist.

**Revendications**

1. Procédé d'évaluation d'un état physiologique d'un tissu corporel, le procédé comprenant :

le repositionnement d'un ou plusieurs transducteurs à ultrasons en mode A (150) sur l'épiderme d'un patient de manière à générer des signaux représentant une distance en ligne droite desdits un ou plusieurs transducteurs à ultrasons (150) à une surface osseuse du patient ;

le suivi du repositionnement desdits un ou plusieurs transducteurs à ultrasons en trois dimensions à l'aide d'au moins une unité de mesure inertielle (170) de manière à générer des données de position 3D d'un os ;

la mise en corrélation des signaux représentant la distance en ligne droite desdits un ou plusieurs transducteurs à ultrasons (150) à la surface osseuse du patient et le changement des données de position 3D de manière à générer une carte 3D comportant une pluralité de points 3D correspondant à des points sur la surface osseuse ;

la superposition de la carte 3D sur un modèle osseux par défaut et la réalisation d'un processus de déformation conduisant à un modèle 3D virtuel spécifique au patient de l'os ;

l'affichage visuel du modèle 3D virtuel de l'os.

2. Procédé selon la revendication 1, ledit modèle osseux par défaut comprenant un modèle osseux généralisé créé à partir de connaissances statistiques d'une vaste population de bases de données d'os.

3. Procédé selon la revendication 2, ledit modèle osseux généralisé étant sélectionné sur la base d'au moins un paramètre parmi le genre, la race, la taille et l'âge du patient.

4. Procédé selon l'une quelconque des revendications 1 à 3, ladite articulation étant déplacée à travers une plage de mouvement tout en repositionnant lesdits un ou plusieurs transducteurs à ultrasons (150) sur l'épiderme du patient de manière à générer les signaux représentant une distance en ligne droite desdits un ou plusieurs transducteurs à ultrasons (150) à une surface osseuse du patient.

5. Procédé selon l'une quelconque des revendications précédentes :

ledit os comprenant un premier os d'une articulation ;

ledit procédé étant répété pour un second os de l'articulation ; et

ledit affichage visuel simultané des modèles 3D virtuels des premier et second os se déplaçant en 3D sur un terminal visuel.

6. Procédé selon l'une quelconque des revendications précédentes, ladite au moins une unité de mesure inertielle (170) comprenant une pluralité d'accéléromètre, de gyroscopes et de magnétomètres.

7. Procédé selon l'une quelconque des revendications précédentes, ledit repositionnement desdits un ou plusieurs transducteurs à ultrasons (150) sur l'épiderme du patient comprenant le repositionnement d'une tige sur l'épiderme du patient, lesdits un ou plusieurs transducteurs à ultrasons étant montés de manière fixe sur la tige, et ladite au moins une unité de mesure inertielle (170) étant montée de manière fixe sur la tige.

FIG. 1
(PRIOR ART)

FIG. 2
(PRIOR ART)

```
┌─────────────────────────────┐
│      KNEE JOINT BONES 3D      │
│     MODELS RECONSTRUCTION     │
└─────────────────────────────┘
        │                │
        ▼                ▼
┌────────────────┐  ┌────────────────────┐
│  MOTION SOUND  │  │ KINEMATICS TRACKING │
│   DETECTION    │  │                     │
└────────────────┘  └────────────────────┘
        │                │
        ▼                ▼
    ┌─────────────────────────────┐
    │  AUTOMATIC ANALYSIS OF THE   │
    │    KINEMATICS AND SOUND      │
    └─────────────────────────────┘
                 │
                 ▼
    ┌─────────────────────────────┐
    │  AUTOMATIC RECOGNITION OF    │
    │        KNEE INJURY           │
    └─────────────────────────────┘
```

FIG. 3

FIG. 4

FIG. 5

ELECTRONIC
COMPONENTS

134

130

130

STRAIN SENSING ARRAY

132

136

FIG. 6

FIG.7

170, 200

```
CLOCK          BASEBAND PULSE              ⊗         ▷        BANDPASS
CRYSTAL    →     GENERATOR        →                              FILTER
```

OSCILLATOR

ANTENNA

## FIG. 8

```
ANTENNA  →  BANDPASS   →   ▷   →   ⊗   →   LPF
              FILTER
```

172

OSCILLATOR

SAMPLING        CLOCK
MIXER      →    CRYSTAL

ADC

DIGITAL SIGNAL
PROCESSING

CPU

## FIG. 9

FIG. 10

FIG. 11

FIG. 12

EP 3 968 220 B1

FIG. 13

EP 3 968 220 B1

FIG. 14

FIG. 15

ECHOMORPHING

DIAGNOSIS

ACCELEROMETERS

LOCATION OF
ACCELEROMETERS

FLUOROSCOPY TO CONSTRUCT
A MODEL TO DEFINE LOCATION
OF ACCELEROMETERS

FIG. 16

110

W$_{ij}$

KINEMATIC FEATURES

SOUND FEATURES

INPUT LAYER

HIDDEN LAYER

OUTPUT LAYER

NORMAL

ACLD

DEGENERATIVE

MENISCUS

FIG. 17

NORMAL & DEGENERATIVE SUBJECTS

SOUND FEATURES EXTRACTION

KINEMATIC FEATURES EXTRACTION

FORCE FEATURES EXTRACTION

FEATURES

TRAINING SET

TESTING SET

TRAINING

TESTING

NEURAL NETWORK

TRAINED NEURAL NETWORK

FIG. 18

FIG. 19

**EP 3 968 220 B1**

**Patent documents cited in the description**

- US 20070249967 A

- US 2009018445 A